# EUROPÄISCHE PATENTSCHRIFT

(11) **EP 3 124 447 B1**
(45) Veröffentlichungstag und Bekanntmachung des Hinweises auf die Patenterteilung: **23.05.2018**
(21) Anmeldenummer: 16180870.4
(22) Anmeldetag: 22.07.2016
(51) Int. Cl.: C03C 3/11, C03C 4/20, A61J 1/00

(54) **BOR-ARMES, ZIRKONIUM-FREIES NEUTRALGLAS MIT OPTIMIERTEM ALKALIVERHÄLTNIS**
LOW-BORON, ZIRCONIUM FREE NEUTRAL GLASS WITH OPTIMIZED ALKALINE RATIO
VERRE NEUTRE SANS ZIRCONIUM, PAUVRE EN BORE AYANT UN RAPPORT EN ALCALIN OPTIMISE

(30) Priorität: 29.07.2015 DE 102015214431
(43) Veröffentlichungstag der Anmeldung: 01.02.2017
(73) Patentinhaber: Schott AG, 55122 Mainz (DE)
(72) Erfinder: KASS, Christof, 95643 Tirschenreuth (DE); TRATZKY, Stephan, 92660 Neustadt/Wn. (DE); MÄNNL, Reinhard, 95666 Mitterteich (DE); EICHHOLZ, Rainer, 60323 Frankfurt am Main (DE)

(56) Entgegenhaltungen:
- DE-A1- 10 025 465
- DE-A1- 10 027 699
- DE-A1- 10 120 475
- DE-A1-102010 029 975
- DE-C1- 4 430 710
- US-A1- 2003 087 745

## Beschreibung

Die Erfindung betrifft ein Zirkonium-freies Neutralglas mit niedrigem Boroxidgehalt und einer guten hydrolytischen Beständigkeit zur bevorzugten Verwendung im Pharmabereich.

### Hintergrund der Erfindung

Im Pharmabereich und für diagnostische und kosmetische Anwendungen kommt Glas als Verpackungsmaterial im großen Umfang zur Anwendung. Insbesondere dessen spezielle Materialeigenschaften, wie hohe Transparenz, gute mechanische Eigenschaften, geringe Permeabilität, in Verbindung mit der guten chemischen Beständigkeit, sind entscheidend für die Beibehaltung der Qualität von z.B. Arzneimitteln und ihrer Wirksamkeit.

Behältnisse für pharmazeutische, therapeutische, diagnostische sowie kosmetische Zwecke sind in der Regel in direktem Kontakt mit dem enthaltenen Wirkstoff und unterliegen daher strengen Anforderungen. Die Behältnisse, welche beispielsweise als Fläschchen, Ampullen, Spritzen oder Karpulen ausgeführt sein können, werden deshalb auch als Primärpackmittel bezeichnet. Während der Lagerung der Wirkstoffe in den Primärpackmitteln sollen möglichst kein Verlust und keine Veränderung der Wirkstoffe auftreten. Die Qualität des Inhalts darf durch den direkten Kontakt mit dem Primärpackmittel nicht in einer Art und Weise verändert werden, die überprüfte und vorgegebene Grenzwerte überschreitet. In jeden Fall muss sichergestellt sein, dass das Glasmaterial keine Substanzen in Mengen abgibt, welche die Stabilität des Wirkstoffs beeinträchtigt oder gar toxisch oder in sonstiger Weise schädlich für einen Anwender sein könnten.

Arzneimittel und andere Wirkstoffe werden meist in gelöster Form, insbesondere auf wässriger Basis weiter verarbeitet und gelagert. Eine besonders wichtige Eigenschaft eines Primärpackmittels aus Glas ist daher die Beständigkeit der Innenoberfläche, die mit dem gelösten Wirkstoff unmittelbar in Berührung kommt und davon angegriffen werden kann. Dadurch können diverse Ionen aus der Glasoberfläche herausgelöst werden und den darin befindlichen Inhalt und Wirkstoff negativ beeinflussen. Um Wirkungsverluste und insbesondere gesundheitliche Risiken auszuschließen, sollten daher generell so wenig wie möglich Ionen aus dem Glas herausgelöst werden. Da Lösungsmittel auf wässriger Basis am gebräuchlichsten sind, ist insbesondere die hydrolytische Beständigkeit eine wichtige Anforderung für die Verwendung eines Glases als Primärpackmittel. Für saure oder basische Lösungen sind entsprechende Anforderungen an die Säurebeständigkeit und Laugenbeständigkeit des Packmittelmaterials zu beachten.

Die hydrolytische Klasse ist auch Grundlage für die Einteilung von Glasarten für pharmazeutische Verwendungen nach dem europäischen Arzneibuch. Gebräuchliche Gläser werden dabei in Klassen unterschieden. Sogenannte Typ I Gläser gehören der hydrolytischen Klasse 1 an und werden auch als Neutralglas bezeichnet. Dazu gehören unter anderem auch die Borosilikatgläser, die wesentliche Mengen an Bor-, Aluminium- oder Erdalkalioxiden enthalten.

Es ist bekannt, dass eine Beigabe oder Erhöhung des Anteils von Boroxid häufig zu einer Verbesserung der hydrolytischen Beständigkeit, einer Erniedrigung der Viskosität der Glasschmelze und zu einer Verringerung des Ausdehnungskoeffizienten führt. Da diese Eigenschaften insbesondere für eine Verwendung als Primärpackmittel besonders willkommen sind, enthalten die bekannten Gläser verhältnismäßig hohe Boroxidanteile (bis 20 %). Bor bindet die vorhandenen Alkaliionen fester in die Glasstruktur ein. Aufgrund der Entdeckung der Gesundheitsschädlichkeit von Boroxid ist dieses Konzept jedoch nicht mehr vertretbar. Boroxid steht als isolierte Substanz in dem Verdacht, fruchtschädigend zu sein. Besonders relevant ist dies für den Herstellprozess eines Glases, da beim Umgang mit Boroxid als einer Ausgangskomponente aufwendige Arbeitsschutzmaßnahmen erforderlich sind, welche die Herstellkosten des Endproduktes verteuern. Aus einem Glas herausgelöste Borkomponenten könnten einen toxikologisch bedenklichen Einfluss auf Lebewesen haben.

Es sind bereits Boroxid-arme (Neutral-) Gläser bekannt.

In der DE 4430710 C1 wird ein Borsäure-armes Borosilikatglas mit hoher chemischer Beständigkeit mit der nachfolgenden Zusammensetzung in Gew.-% auf Oxidbasis offenbart:

| | |
|---|---|
| SiO₂ | > 75 |
| B₂O₃ | 1 - < 9 |
| Al₂O₃ | 3 - 6 |
| Li₂O | 0 - 4 |
| Na₂O | 0 - 8 |
| K₂O | 0 - 6 |
| MgO | 0 - 3 |
| CaO | 0 - 3 |
| BaO | 0 - 2 |
| SrO | 0 - 2 |
| ZnO | 0 - 3 |
| ZrO₂ | 0 - 3 |
| SnO₂ | 0 - 3 |
| SnO | 0 - 3 |
| TiO₂ | 0 - 2 |
| CeO₂ | 0 - 2 |
| Fe₂O₃ | 0 - 1 |
| mit | |
| SiO₂ +B₂O₃ | > 83 |
| SiO₂ : B₂O₃ | > 8 |
| SiO₂+Al₂O₃+ZrO₂ | > 83 |
| Li₂O+Na₂O+K₂O | 5 - 10 |
| MgO+CaO+BaO+SrO+ZnO | ≤ 3. |

Laut Angaben in der DE 4430710 C1 erreichen die in den Beispielen genannten Gläser die hydrolytische Klasse HBK1 (nach der veralteten DIN 12111). Im Rahmen der Erfindung durchgeführte Bestimmungen nach der moderneren Methode gemäß der United States Pharmacopeia (USP, siehe unten) ergaben jedoch, dass die offenbarten Glasbeispiele, bezogen auf den Grenzwert der hydrolytischen Klasse 1, nur im mittleren Bereich liegen und daher keine wettbewerbsfähigen Pharmagläser sind. Die chemische Beständigkeit wird bei den beschriebenen Glasbeispielen durch hohe Gehalte von Zirkoniumoxid/ZrO₂ (bis 1,6 Gew.-%) und/oder relativ hohe Boroxid-Gehalte (bis 8,9 Gew.-%) erreicht.

Als Alkali-Anteil wird in den beispielhaft angegebenen Glaszusammensetzungen fast ausschließlich Natriumoxid und Lithiumoxid angegeben. Dabei liegt der Natriumoxidgehalt in den Beispielen oberhalb des erfindungsgemäßen Bereiches. Kaliumoxid kommt nur im Beispiel 5 zum Einsatz, allerdings hier in Verbindung mit einem deutlich über dem erfindungsgemäßen Grenzwert liegenden Gehalt an Lithiumoxid und einem hohen Zirkoniumoxidgehalt. Messungen in Rahmen dieser Erfindung haben ergeben, dass eine derartige Zusammensetzung Gläser mit relativ schlechten Werten innerhalb der hydrolytischen Klasse HBK1 ergeben (hohe Alkaliionenabgabe).

Gemäß der Offenbarung der DE102010029975 A1 wird ein Boroxid-armes Borosilikatglas mit folgender Zusammensetzung (in Gew-% auf Oxidbasis) besch rieben:

| | |
|---|---|
| SiO₂ | 70 - 79 |
| B₂O₃ | 0 - < 5 |
| Al₂O₃ | 1 - < 5 |
| ZrO₂ | 0,5 - < 5 |
| TiO₂ | 0,5 - 6 |
| Na₂O | 1 - 6 |
| K₂O | 3 - 8 |
| Li₂O | 0 - 0,5 |

mit

| | |
|---|---|
| SiO₂ + B₂O₃ | < 83 |

Zur Erzielung der hydrolytischen Beständigkeit enthalten die dort beschriebenen Neutralgläser zwingend Zirkoniumoxid und Titanoxid in aufeinander abgestimmten Massenverhältnissen. Dabei wird der Zirkoniumanteil auch an den Kaliumanteil gekoppelt, um die physikalischen und chemischen Eigenschaften der Zusammensetzungen einzustellen. Die guten chemischen Eigenschaften dieser Gläser werden auf den erforderlichen Gehalt von Zirkonium-, Titan- und Kaliumoxid und deren Zusammenwirken im Glas zurückgeführt.

Es ist bekannt, dass Zirkoniumoxid als stabilisierender Zusatz die chemische Beständigkeit von Gläsern fördert. Dafür kommt es in der DE 4430710 C1 und der DE 102010029975 A1 gezielt zum Einsatz. Zur Erhöhung der hydrolytischen Stabilität wird es in der DE 102010029975 A1 als zwingend erforderlicher Bestandteil in Kombination mit Titanoxid und in Kombination mit Kaliumoxid in speziellen Mengenverhältnissen verwendet zur Erzielung eines synergistischen Effektes. Zirkonium ist als Bestandteil eines Neutralglases im pharmazeutischen Bereich jedoch problematisch, da diese Gläser einer speziellen Zulassung bedürfen und Zirkonium ein unerwünschter Bestandteil sein kann, weil es unter anderem geringe Mengen an Uranoxiden und anderen radioaktiven Stoffen (z.B. Thorium-Verbindungen) aufweisen kann.

Der vorliegenden Erfindung liegt die Aufgabe zugrunde, eine Glaszusammensetzung vorzuschlagen, die im hohen Maße die Anforderungen für Gläser erfüllt, die als Primärpackmittel, insbesondere im Pharmabereich eingesetzt werden sollen. Dazu gehört im Besonderen auch eine gute hydrolytische Beständigkeit, d.h. eine geringe lonenabgabe an ein wässriges Medium.

### Beschreibung der Erfindung

Erfindungsgemäß wird die Aufgabe durch die Glaszusammensetzung des Anspruchs 1 gelöst.

Das bevorzugt zur Verwendung im Pharmabereich geeignete, Zirkonium (Zr)-freie Neutralglas weist die nachfolgende Zusammensetzung in Gew.-% auf oder besteht daraus:

| | |
|---|---|
| SiO₂ | 72 - 82 |
| B₂O₃ | 3 - 8 |
| Al₂O₃ | 5 - 8 |
| Na₂O | 2,5 - 5,5 |
| K₂O | 3,6 - 8,4 |
| Li₂O | 0 - 0,7 |
| MgO | 0 - 0,7 |
| CaO | 0 - 0,4 |
| TiO₂ | 0 - 5 |

mit

| | |
|---|---|
| Li₂O+ Na₂O+ K₂O | 6,8 - 14,6 Gew.-% |
| K₂O/Na₂O-Verhältnis | 0,7 - 3,4 |

Es versteht sich, dass die Komponenten in der Glaszusammensetzung derart ausgewählt werden, dass sich insgesamt 100 Gew.-% ergeben. Sofern nichts anderes angegeben ist, beziehen sich alle Anteilsangaben auf die fertigen Glaszusammensetzungen im geschmolzenen Glas in Gew.-% basierend auf den jeweiligen Oxiden.

Das erfindungsgemäße Neutralglas ist der hydrolytischen Klasse 1 zuzuordnen und weist auch eine gute Säure- und Laugenbeständigkeit auf. Dabei enthält es erfindungsgemäß kein zugesetztes Zirkonium. Zirkonium-frei bedeutet dabei, dass keine Zirkonium-haltige Verbindung dem Ausgangsgemisch beigemengt wird. Verunreinigungen können im Glas vorliegen. Durch die Erfindung wird somit ein Glas zur Verfügung gestellt, das im Unterschied zu anderen für pharmazeutische Verpackungen vorgeschlagenen Primärpackmitteln bezüglich dieses Inhaltsstoffes keiner gesonderten Zulassung bedarf. - Ferner enthält das erfindungsgemäße Glas verhältnismäßig wenig Boroxid, insbesondere im Vergleich zu Gläsern, die aktuell für die Lagerung und Verpackung von Injektabilia häufig verwendet werden (z.B. FIOLAX®von SCHOTT). Als Behältnisse für Primärpackmittel kommen insbesondere Fläschchen, Ampullen, Karpulen und Spritzen zum Einsatz. Diese werden üblicherweise durch Heißumformung aus einem Glasrohr hergestellt. Das erfindungsgemäße Glas eignet sich auf Grund seiner technischen und physikalischen Eigenschaften für die Glasrohrherstellung. Durch den geringen Boroxidgehalt verdampfen bei der Heißformgebung und Umformung deutlich weniger Borate als bei den üblicherweise eingesetzten Gläsern. Insbesondere bei der Weiterverarbeitung zu Fläschchen kann eine starke Borabdampfung an Teilen der Innenflächen Korrosionseffekte hervorrufen, die im späteren Einsatz solchermaßen geschädigter Fläschchen zu einer erhöhten Delaminationsgefahr führen, darunter versteht man die Ablösung makroskopischer Glasflitter von der Wandung, und zu einer erhöhten Alkaliabgabe von Glasbehältnissen gemäß ISO 4802-1 und -2 führen. Diese nachteiligen Effekte sind bei einem erfindungsgemäßen Neutralglas reduziert.

Im Rahmen der Erfindung wurde in unerwarteter Weise festgestellt, dass aus der gezielten Mengenwahl der Alkalioxide von Natrium, Kalium und ggf. Lithium in der Glaszusammensetzung sowie aus der Einstellung eines bestimmten Verhältnisses von Kaliumoxid zu Natriumoxid eine nicht vorhersehbare hydrolytische Beständigkeit des erzeugten Neutralglases resultiert, und zwar ohne die Beimengung des bei den Gläsern des Standes der Technik erforderlichen Zirkoniumoxides. Überraschend ist auch, dass sich dieser Effekt (feste Einbindung der Alkaliionen in die Glasstruktur) einstellt, obwohl das erfindungsgemäße Glas einen im Vergleich zu üblichen Borosilikatgläsern verhältnismäßig niedrigen Boroxidgehalt aufweist.

Neben dem genannten und unten detailliert beschriebenen K₂O/Na₂O-Verhältnis, das im angegebenen Bereich einzustellen ist, spielen auch die anderen Komponenten der Glaszusammensetzung eine Rolle.

SiO₂ als Hauptbestandteil der Glaszusammensetzung ist ein wesentlicher Glasbildner und bewirkt, dass das Glas ausreichend stabil und beständig ist. Der Anteil liegt erfindungsgemäß im Bereich von 72 bis 82 Gew.-%. Vorteilhaft kann er auch im Bereich von 75 bis 81 Gew.-%, bevorzugt im Bereich von 77 bis 80 Gew.-% liegen. In der erfindungsgemäßen Zusammensetzung ist wenigstens 72 Gew.-% SiO₂ vorhanden. Eine kleinere Menge würde die hydrolytische Stabilität des Glases beeinträchtigen. Die Obergrenze beträgt erfindungsgemäß 82 Gew.-%. Durch höhere Anteile an SiO₂ würden sich die Schmelztemperatur und die Verarbeitungstemperatur des Glases (d.h. die Temperatur, bei der das Glas eine für die Verarbeitung geeignete Viskosität aufweist, die in etwa bei 10⁴ dPas liegt) zu sehr erhöhen. Die SiO₂-Untergrenze kann bei einer vorteilhaften Glaszusammensetzung auch bei 75 Gew.-%, bevorzugt bei 77 Gew.-% liegen. Als SiO₂-Obergrenze kann bei vorteilhaften Ausführungsformen 81 Gew.-%, bevorzugt 80 Gew.-% gewählt werden.

B₂O₃ ist wie SiO₂ ein Glasbildner und wird in Gläsern eingesetzt zur Erzielung einer guten hydrolytischen Beständigkeit, zur Viskositätserniedrigung und zur Verringerung des Ausdehnungskoeffizienten. Erfindungsgemäß liegt Boroxid in der Glaszusammensetzung im Bereich von 3 bis 8 Gew.-%, bevorzugt im Bereich von 3 bis 6,5 Gew.-%, besonders bevorzugt im Bereich von 3,5 bis 5,5 Gew.-% vor. Da Boroxid die Alkaliionen in die Glasstruktur einbindet, ist erfindungsgemäß wenigstens 3 Gew.-% B₂O₃ in der Glaszusammensetzung enthalten. Der erfindungsgemäße Anteil von B₂O₃ im Glas ist auf maximal 8 Gew.-% begrenzt, um die oben erwähnte Verdampfung von Boraten, insbesondere bei der Bodenformung der Behältnisse, und die damit verbundenen Probleme gering zu halten. Bei manchen Gläsern kann als eine vorteilhafte Untergrenze für Boroxid auch 3,5 Gew.-% gewählt werden. Vorteilhafte Ausführungsformen der Erfindung enthalten maximal nur 6,5 Gew.-% B₂O₃, besonders bevorzugte Ausführungsformen nur 5,5 Gew.-% B₂O₃ Im Vergleich zu herkömmlichen, im Vertrieb befindlichen Pharmagläsern vom Typ I (z.B. Fiolax® von SCHOTT) ist das erfindungsgemäße Glas im Boroxidgehalt reduziert, bevorzugt stark reduziert, und weist dennoch im Vergleich eine verbesserte hydrolytische Beständigkeit auf.

Der Gehalt an Al₂O₃ in der erfindungsgemäßen Glaszusammensetzung beträgt 5 bis 8 Gew.-%, bevorzugt 5 bis 7,5 Gew.-%. Aluminium wird als Netzwerkbildner eingesetzt und zieht die gesamte Glasstruktur zusammen. Die Lücken im Glasnetzwerk werden für die Kationen dadurch kleiner. Die engere Struktur hat bei Auslaugung durch wässrige Lösungen eine geringere Alkaliabgabe zur Folge. Außerdem verbessert der Zusatz von Aluminiumoxid die Entglasungseigenschaften (siehe unten). Erfindungsgemäß sollte ein Al₂O₃-Mindestgehalt von 5 Gew.-% nicht unterschritten werden, damit es bei der Formgebung (z.B. während des Ziehprozesses eines Glasrohres) nicht zu einer störenden Kristallbildung (auch Entglasung genannt) kommt. Da Aluminium die Schmelztemperatur und die Verarbeitungstemperatur der Glaszusammensetzung erhöht, ist erfindungsgemäß maximal 8 Gew.-% Aluminiumoxid vorgesehen. Vorteilhafte Ausführungsformen können auch maximal 7,5 Gew.-% Al₂O₃ enthalten. Der Mengenanteil von Aluminiumoxid in der Glaszusammensetzung hängt auch von dem gewählten Verfahren ab, mit dem das erschmolzene Glas in Form gebracht (z.B. als Rohr gezogen) wird.

Oxide von Kalium, Natrium und ggf. Lithium spielen eine Rolle für die Einstellung der Wärmedehnung (thermischer Ausdehnungskoeffizient), verbessern die Schmelzbarkeit des Glases und verringern die Viskosität. Erfindungsgemäß liegt die Summe der Alkalioxide im Bereich von 6,8 bis 14,6 Gew.-%. In vorteilhaften Ausführungen kann die Summe der Alkalioxide auch 6,8 bis 13,3 Gew.-%, bevorzugt 7 bis 12,6 Gew.-% betragen. Die Untergrenze der Alkalioxid-Summe von 6,8 Gew.% sollte nicht unterschritten werden, da sich ansonsten die Schmelzbarkeit der erhaltenen Gläser zu stark verschlechtert. Oberhalb der Alkalioxid-Summe von 14,6 Gew.-% verschlechtert sich die chemische Beständigkeit der Gläser. Bei einer vorteilhaften Glaszusammensetzung kann die Untergrenze für die Summe der Alkalioxide auch 7 Gew.-% betragen. Im Hinblick auf die Obergrenze können Alkalioxide vorteilhaft auch mit einer Summe von 13,3 Gew.-%, bevorzugt mit einer Summe von 12,6 Gew.-% eingesetzt werden.

Erfindungsgemäß kann der Glaszusammensetzung Lithiumoxid mit einem Anteil von 0 bis 0,7 Gew.-% beigefügt sein. Bevorzugt ist ein Li₂O-Gehalt von 0 bis 0,5 Gew.-%. Li₂O kann bei manchen Glaszusammensetzungen eingesetzt werden, um die Verarbeitungstemperatur zu senken. Da Lithium einen sehr kleinen lonenradius hat, besteht die Gefahr der Auslaugung aus den Lücken der Glasstruktur. Daher sollte der maximale Li₂O-Anteil von 0,7 Gew.-% nicht überschritten werden, um die hydrolytische Beständigkeit nicht abzusenken.

Vorteilhaft kann es auch sein, wenn die Glaszusammensetzung maximal 0,5 Gew.-% Li₂O enthält. Vorzugsweise wird ganz auf die Zugabe von Lithium verzichtet.

Na₂O wird in Gläsern eingesetzt, weil Natrium in Verbindung mit Aluminium deren hydrolytische Beständigkeit verbessert. Außerdem wird durch Natrium die Schmelztemperatur gesenkt. Der Natriumoxid-Gehalt liegt erfindungsgemäß im Bereich von 2,5 bis 5,5 Gew.-%, bevorzugt im Bereich von 2,8 bis 4,8 Gew.-%. Ein erfindungsgemäßes Glas enthält somit mindestens 2,5 Gew.-% Natriumoxid. Die Obergrenze für Na₂O von maximal 5,5 Gew.-% sollte nicht überschritten werden, da sich die chemische Beständigkeit ansonsten verschlechtert. Es kann vorteilhaft sein, mindestens 2,8 Gew.-% Na₂O im Glas vorzusehen. Vorzugsweise kann die Obergrenze für Natriumoxid auch 4,8 Gew.-% betragen.

Der K₂O-Gehalt der erfindungsgemäßen Glaszusammensetzung beträgt 3,6 bis 8,4 Gew.-%. In einer vorteilhaften Ausführungsform liegt er im Bereich von 4 bis 8 Gew.-%, bevorzugt im Bereich von 4,2 bis 7,8 Gew.-%. Kalium bewirkt eine gute hydrolytische Beständigkeit und verbessert die Entglasungseigenschaften des Glases. Um diesen Effekt zu erzielen, liegt die erfindungsgemäße Untergrenze für Kaliumoxid bei 3,6 Gew.-%. Erfindungsgemäß kommt nicht mehr als 8,4 Gew.-% K₂O zum Einsatz, da sich darüber die chemische Beständigkeit des Glases verschlechtert. Die K₂O-Untergrenze kann bei einer vorteilhaften Glaszusammensetzung auch bei 4 Gew.-%, bevorzugt bei 4,2 Gew.-% liegen. Als Obergrenze kann bei vorteilhaften Ausführungsformen 8 Gew.-% K₂O, bevorzugt 7,8 Gew.-% K₂O gewählt werden.

Im Rahmen der Erfindung wurde festgestellt, dass nicht nur die einzelnen, vorstehend beschriebenen Mengen an Natriumoxid, Kaliumoxid und ggf. Lithiumoxid sowie deren oben beschriebene Gesamtsumme im Glas für die chemische Beständigkeit eine wichtige Rolle spielen. Darüber hinaus wird erfindungsgemäß ein spezielles Verhältnis von Kaliumoxid und Natriumoxid in der Glaszusammensetzung eingestellt. Erfindungsgemäß liegt im Glas ein K₂O/Na₂O-Verhältnis von 0,7 bis 3,4 vor. Überraschenderweise werden die Alkaliionen bei Einstellung eines solchen Verhältnisses fest in die Glasstruktur eingebunden, auch wenn die Glaszusammensetzung kein Zirkoniumoxid (ZrO₂) und nur eine relativ geringe Menge Boroxid enthält. Die derart zusammengesetzten Neutralgläser weisen eine sehr gute chemische Beständigkeit, insbesondere eine ausgezeichnete hydrolytische Beständigkeit auf. Sie haben nicht nur die hydrolytische Klasse 1, sondern liegen innerhalb dieser Klasse im unteren Viertel bis Drittel, bezogen auf den oberen Grenzwert (siehe Tabelle 1). Dies ist insbesondere für Verwendungen im Pharmabereich von Bedeutung, da ein Herauslösen von Ionen aus dem Glas gesundheitliche Risiken bergen kann. Die erfindungsgemäßen Gläser sind diesbezüglich deutlich besser als die aus dem Stand der Technik bekannten Boroxid-armen, Zirkoniumhaltigen Neutralgläser. Auch in Bezug auf die Säurebeständigkeit (bestimmt nach DIN 12116) und die Laugenbeständigkeit (bestimmt nach ISO 695) weisen die erfindungsgemäßen Gläser bessere Werte auf, als bekannte Neutralgläser. Ein K₂O/Na₂O-Verhältnis von <0,7 sollte nicht eingestellt werden, weil sich ansonsten die Natriumabgabe und die Entglasungseigenschaften verschlechtern. Auch sollte die Obergrenze des K₂O/Na₂O-Verhältnisses von 3,4 eingehalten werden, weil darüber die Verdampfungsverluste von Kalium beim Schmelzen zu hoch werden.

In vorteilhaften Ausführungsformen liegt ein K₂O/Na₂O-Verhältnis von bevorzugt 0,8 bis 2,9, mehr bevorzugt von 0,9 bis 2,8 vor. Eine Untergrenze des K₂O/Na₂O-Verhältnisses kann vorteilhaft 0,8, bevorzugt 0,9 sein. Eine Obergrenze des K₂O/Na₂O-Verhältnisses kann vorteilhaft 2,9, bevorzugt 2,8 sein.

Eine besonders bevorzugte Glaszusammensetzung weist 3,5 Gew.-% Na₂O und 6,5 Gew.-% K₂O und damit ein K₂O/Na₂O-Verhältnis von 1,86 auf.

Erdalkalioxide können in der Glaszusammensetzung in geringen Mengen enthalten sein. Der CaO-Gehalt im erfindungsgemäßen Glas liegt im Bereich von 0 bis 0,4 Gew.-%. Der MgO-Gehalt im erfindungsgemäßen Glas liegt im Bereich von 0 bis 0,7 Gew.-%. Oberhalb der genannten Grenzen verschlechtert sich die hydrolytische Beständigkeit der Neutralgläser. Bevorzugt werden der Glaszusammensetzung kein CaO und/oder kein MgO beigefügt. Bariumionen lassen sich nur in geringem Maß aus der Glasstruktur auslaugen, sind in der Pharmaindustrie jedoch unerwünscht, da bei bestimmten Füllgütern Ausfällungen vorkommen. Bevorzugt enthält das Neutralglas kein BaO. Eine vorteilhafte Glaszusammensetzung enthält kein Erdalkalioxid, d.h. Erdalkalioxid wird dem Ausgangsgemisch nicht beigemengt. Verunreinigungen können im Glas jedoch vorliegen.

Der TiO₂-Gehalt im erfindungsgemäßen Glas liegt im Bereich von 0 bis 5 Gew.-%. Titanoxid kann die Viskosität des Neutralglases erniedrigen. Zudem schützt es vor UV-Strahlung und verhindert Solarisation (Nachdunkeln durch Lichteinwirkung). Die Obergrenze von 5 Gew.-% TiO₂ sollte nicht überschritten werden, da sich andernfalls die Entglasungseigenschaften des resultierenden Glases verschlechtern würden. Vorteilhafte Ausführungsformen enthalten kein zugesetztes TiO₂

Im Herstellungsprozess für das erfindungsmäße Glas werden dem Gemenge der Ausgangkomponenten in bekannter Weise noch Läutermittel (wie z.B. Antimonoxid, Ceroxid, Zinnoxid, Chloride) und Schmelzbeschleuniger (wie z.B. Fluoride) in bekannten Mengen zugegeben, die -je nach eingesetztem Mittel - noch im fertigen Glas enthalten sein können (z.B. Fluoride 0 bis 1 Gew.%, Chloride 0 bis 0,5 Gew.%).

Durch die erfindungsgemäße spezielle Kombination der Alkalioxide von Natrium, Kalium und ggf. Lithium, sowie eine gezielte Menge an Aluminiumoxid wird ein erfindungsgemäßes Zirkonium-freies, Bor-armes Neutralglas bereitgestellt, das sich neben der ausgezeichneten hydrolytischen Beständigkeit und der sehr guten Laugen- und Säurenbeständigkeit dadurch auszeichnet, dass es kristallisationsstabil ist und keine bzw. nur eine sehr geringe Entglasungsneigung aufweist.

Eine erfindungsgemäße Glaszusammensetzung führt zu einem Neutralglas, das in Rohrform herstellbar (z.B. zu einem Rohr ziehbar) ist und vorteilhaft für die Weiterverarbeitung zu Behältern, wie Ampullen, Karpulen, Spritzen etc. geeignet ist. Alternativ können aus dem Glas auch andere Formen wie z.B. Flachgläser, Glasblöcke etc. erzeugt werden. Das erfindungsgemäße Neutralglas zeichnet sich im Vergleich zu bekannten Gläsern (z.B. DE102010029975 A1) durch weniger Borat-Verdampfung und eine höhere Kristallisationsbeständigkeit aus, so dass es auch mit dem Danner-Verfahren zu einem Rohr ziehbar ist. Selbstverständlich sind auch andere Rohrziehverfahren möglich, beispielsweise das Vello-Ziehverfahren.

Eine vorteilhafte Ausführungsform betrifft ein Zirkonium-freies Neutralglas mit hoher hydrolytischer Beständigkeit, das eine Zusammensetzung aufweist, die in Gew.-% umfasst:

| | |
|---|---|
| SiO₂ | 75 - 81 |
| B₂O₃ | 3 - 6,5 |
| Al₂O₃ | 5 - 7,5 |
| Na₂O | 2,8 - 4,8 |
| K₂O | 4,0 - 8,0 |
| Li₂O | 0 - 0,5 |
| Li₂O+ Na₂O+ K₂O | 6,8 - 13,3 |
| Verhältnis K₂O/Na₂O | 0,8 - 2,9. |

Hinzu kommen übliche Läutermittel in üblichen Mengen.

Eine besonders vorteilhafte Ausführungsform betrifft ein Zirkonium-freies Neutralglas mit hoher hydrolytischer Beständigkeit, das eine Zusammensetzung aufweist, die in Gew.-% umfasst:

| | |
|---|---|
| SiO₂ | 77 - 80 |
| B₂O₃ | 3,5 - 5,5 |
| Al₂O₃ | 5,0 - 7,5 |
| Na₂O | 2,8 - 4,8 |
| K₂O | 4,2 - 7,8 |
| Li₂O+ Na₂O+ K₂O | 7,0 - 12,6 |
| Verhältnis K₂O/Na₂O | 0,9 - 2,8. |

Hinzu kommen übliche Läutermittel in üblichen Mengen.

Die Erfindung bezieht sich auch auf ein gezogenes Rohr, das aus dem erfindungsgemäßen Glas besteht oder dieses umfasst. Ferner bezieht sich die Erfindung auch auf die Verwendung des erfindungsgemäßen Glases zur Herstellung eines gezogenen Rohres.

Gegenstand der Erfindung ist auch die Verwendung eines erfindungsgemäßen Neutralglases im Pharmabereich. Insbesondere kann das Glas für Fläschchen, Spritzen, Ampullen und/oder Karpulen verwendet werden. Ein solches aus dem erfindungsgemäßen Glas hergestelltes Behältnis ist ebenfalls Gegenstand der Erfindung.

Weiterhin kann das erfindungsgemäße Glas aufgrund der chemischen Beständigkeit Verwendung finden als Laborglas (z.B. für ein Laborgerät wie Pipette, Bürette, Becherglas, Reagenzglas, Kolben, Messkolben, Zylinder, Messzylinder, Kühler, Kühlfalle, Trichter, Schale, U-Rohr, Dewargefäß, Thermometer etc.) sowohl im pharmazeutischen Bereich als auch im nichtpharmazeutischen Bereich. Ein aus dem erfindungsgemäßen Glas hergestelltes Laborgerät ist ebenfalls Gegenstand der Erfindung.

Ferner ist Gegenstand der Erfindung die Verwendung eines erfindungsgemäßen Neutralglases als Primärpackmittel, vorzugsweise als Primärpackmittel für Arzneimittel, insbesondere für wässrige oder wasserhaltige Arzneimittel.

Der Begriff "Primärpackmittel" ist im Rahmen der Erfindung breit zu verstehen und umfasst jede Art, Größe und Form von Glasbehältnis im weitesten Sinne. Hierunter fällt jede Art Hohlkörper aus Glas, der verschließbar ist und im Pharmabereich eingesetzt werden kann (z.B. Flaschen, Fläschchen, Vial, Phiolen, Ampullen, Karpulen, Spritzen etc.).

Im Rahmen der Erfindung wird unter "wässrigem oder wasserhaltigem Arzneimittel" jede Art von Arzneimittelwirkstoff, auch Mischungen von mehreren Arzneimittelwirkstoffen verstanden, der/die Wasser aufweist/aufweisen.

Unter "wässrigem Arzneimittel" wird ein Arzneimittel verstanden, dessen Hauptbestandteil Wasser darstellt. Zum Beispiel kann Wasser den Hauptbestandteil des Dispergiermediums darstellen, in dem der/die Arzneimittelwirkstoff(e) verteilt vorliegt/vorliegen. Beispielsweise kann eine wässrige Lösung vorliegen, in der der Arzneimittelwirkstoff gelöst vorliegt, wie beispielsweise eine Injektionslösung.

Unter "wasserhaltigem Arzneimittel" wird auch ein Arzneimittel verstanden, dessen Hauptbestandteil nicht Wasser darstellt. Beispielsweise kann dies ein wasserhaltiges Medium, beispielsweise eine Lösung darstellen, das einen Anteil an Wasser und andere Lösungsmittel, wie z.B. Alkohol aufweist, worin mindestens ein Arzneimittelwirkstoff dispergiert oder gelöst vorliegt.

Das Arzneimittel kann in flüssiger Form vorliegen. Dies muss aber nicht in jedem Fall so sein. Es gibt auch halbfeste oder feste Arzneimittel-Formulierungen (z.B. ein Pulver), die in erfindungsgemäßen Primärpackmitteln vorliegen können.

Die Gläser der vorliegenden Erfindung weisen vorteilhafte Eigenschaften auf, die zu ihrer fehlerfreien und kostengünstigen Massenproduktion, insbesondere zur Herstellung von Pharmaprimärpackmitteln, erforderlich sind. Sie erfüllen die notwendigen Voraussetzungen an das Schmelzverhalten, die Kristallisationsstabilität, die Läutereigenschaften, die Verarbeitbarkeit und die chemische Beständigkeit (insbesondere die hydrolytische Beständigkeit). Somit sind die erfindungsgemäßen Neutralgläser für die Herstellung von Pharmaprimärpackmitteln besonders geeignet. Sie sind gut schmelzbar und lassen sich gut zu Rohren ziehen. Pharmazeutische Behältnisse wie Primärpackmittel aus Glas werden üblicherweise durch Heißumformung aus Glasrohren gefertigt, so dass der Herstellung von Glasrohren eine besondere Bedeutung zukommt. Insgesamt sind die erfindungsgemäßen Gläser großtechnisch in ausreichend guter Qualität wirtschaftlich herstellbar.

Die Erfindung bezieht sich auch auf ein Primärpackmittel, dass sich dadurch auszeichnet, dass es aus einem erfindungsgemäßen Neutralglas besteht. Durch die in den erfindungsgemäßen Primärpackmitteln aufbewahrten Arzneimittel, insbesondere wässrige oder wasserhaltige Arzneimittel, beispielsweise Injektionslösungen, wird die Innenfläche des Behältnisses nicht in nennenswerter Art und Weise angegriffen, so dass keine oder nur wenige Ionen aus dem Glas freigesetzt werden.

Da das erfindungsgemäße Neutralglas im Kontakt mit Wasser, Wirkstoffen und/oder Puffersystemen im pH-Bereich von 1 bis 11 chemisch beständig (chemisch inert) ist, eignet es sich hervorragend zur Herstellung von pharmazeutischen Behältnissen (Primärpackmitteln). Ein aus dem erfindungsgemäßen Glas hergestelltes Behältnis kann somit besonders gut für die Lagerung von Wasser, eines Wirkstoffes und/oder einer Pufferlösung im pH-Bereich von 1 bis 11 verwendet werden.

Ein aus dem erfindungsgemäßen Neutralglas gefertigtes pharmazeutisches Behältnis ist vorteilhaft zur Lagerung von Wasser und/oder eines Wirkstoffes und/oder einer Pufferlösung im pH-Bereich von 4 bis 9 geeignet (z.B. 1 molare bzw. 8,4%-ige Natriumbicarbonant-Lösung NaHCO₃ mit einem pH-Wert von ca. 8).

Bevorzugt ist ein aus dem erfindungsgemäßen Neutralglas gefertigtes pharmazeutisches Behältnis geeignet zur Lagerung eines Wirkstoffes und/oder einer Pufferlösung im pH-Bereich von 5 bis 7 (z.B. für einen 10 mmolaren CitratPuffer (pH=6) mit 150 mmolarer NaCl und 0,005%Tween 20 oder für einen 10 mmolaren Phosphat-Puffer (pH=7) mit 150 mmolarer NaCl und 0,005% Tween 20) und/oder zur Lagerung von Wasser, insbesondere von Wasser für Injektionszwecke (z.B. Satorius-Reinstwasser, durch 0,2 µm Filter gespühlt, mit 18,2 MΩ x cm Widerstand).

Gegenstand der Erfindung ist somit auch ein aus dem erfindungsgemäßen Neutralglas hergestelltes Primärpackmittel, das mindestens eine Komponente enthält, ausgewählt aus der Gruppe von Wasser, Wirkstoff, Pufferlösung im pH-Bereich 1 bis 11.

Gegenstand der Erfindung ist ferner ein aus dem erfindungsgemäßen Neutralglas hergestelltes Primärpackmittel, das mindestens eine Komponente enthält, ausgewählt aus der Gruppe von Wasser, Wirkstoff, Pufferlösung im pH-Bereich 4 bis 9.

Gegenstand der Erfindung ist weiterhin ein aus dem erfindungsgemäßen Neutralglas hergestelltes Primärpackmittel, das mindestens eine Komponente enthält, ausgewählt aus der Gruppe von Wasser, Wirkstoff, Pufferlösung im pH-Bereich 5 bis 7.

Gegenstand der Erfindung ist darüber hinaus ein aus dem erfindungsgemäßen Neutralglas hergestelltes Primärpackmittel, das Wasser für Injektionszwecke enthält.

Nachfolgend wird die vorliegende Erfindung anhand von Beispielen erläutert, welche die erfindungsgemäße Lehre veranschaulichen, diese aber nicht beschränken sollen.

### Beispiele

Es ist zu beachten, dass die Mengen der Komponenten, die in den nachfolgenden Tabellen angeben sind, Mengen sind, die im geschmolzenen Glas vorliegen. Dem Fachmann ist bekannt, dass manche Komponenten (z.B. Boroxid, (Borsäure, Alkaliborat)) eine Verdampfungstendenz im Herstellungsprozess des Glases und bei Umformprozessen haben. Bei diesen Komponenten ist die in der Ausgangszusammensetzung vorhandene Menge somit größer als später im geschmolzenen Glas. Ferner ist dem Fachmann geläufig, dass die erforderlichen Schmelztemperaturen und Temperaturen während der Verarbeitung das Ausmaß der Verdampfung beeinflussen, so dass er die einzusetzenden Mengen gezielt berechnen kann. Das Verdampfen der flüchtigen Komponenten führt zu größeren Mengen an verbleibenden nichtflüchtigen Komponenten im geschmolzenen Glas.

Aus dem erfindungsgemäßen Glaszusammensetzungsbereich wurden sieben Ausführungsbeispiele exemplarisch ausgewählt (siehe Tabelle 1). Dabei umfasste das Herstellungsverfahren die Schritte: Mischen der Glaskomponenten, Schmelzen der Glaskomponenten und Läutern der Glasschmelze. Anschließend wurden Rohre bzw. Rohrabschnitte hergestellt (mit dem Danner-Verfahren gezogen).

An den erzeugten Proben der Glasbeispiele wurden die physikalischen und chemischen Eigenschaften bestimmt.

Die bei Neutralgläsern für den pharmazeutischen Bereich besonders wichtige Eigenschaft der hydrolytischen Beständigkeit und die Klassifizierung wurden nach dem normierten "Glass Grains Test" gemäß der United States Pharmacopeia (USP 38, Kap. 660 "Containers-Glass") bestimmt.

Die Einteilung von Gläsern nach USP 38 in 3 Typen (Typ I, Typ II, Typ III) erfolgt gemäß folgendem Verfahren:
Reinigen des zu testenden Ausgangsglases, zerkleinern im Mörser oder in der Kugelmühle, sieben mit drei unterschiedlichen normierten Siebmaschenweiten, mehrfache Reinigung mit Aceton im Ultraschallbad und Trocknung - alles entsprechend einem Ablauf, der in der USP genau vorgegeben ist. Es wird ein Glasgrieß mit einer definierten Korngröße erhalten.

In einem Kolben werden 10 g Glasgrieß mit 50 ml kohlenstoffdioxidfreiem, gereinigtem Wasser versetzt (Glasprobe). Der verschlossene Kolben wird unter speziell vorgegebenen Bedingungen für 30 min (+/- 1 min) bei 121°C (+/- 1°C) autoklaviert und abgekühlt, ebenso eine entsprechende Blindprobe ohne Glasgrieß. Anschließend werden die Blindprobe und die Glasprobe mit 0,05 ml Methylrot-Lösung (die gemäß USP-Vorgaben hergestellt wurde) versetzt und bis zum Farbumschlag von rot zu gelb mit 0,02 M Salzsäure titriert. Nach Abzug des Titrationsvolumens der Blindprobe von dem Titrationsvolumen der Glasprobe wird das Ergebnis in ml Verbrauch von 0,02 M Salzsäure pro Gramm Probe berechnet.

Beträgt das Volumen der verbrauchten 0,02 M Salzsäure pro Gramm Probe maximal 0,1 ml, handelt es sich um Typ I-Gläser, d.h. Gläser die der hydrolytischen Klasse 1 angehören. Gläser, bei denen maximal 0,85 ml 0,02 M Salzsäure pro Gramm Probe verbraucht wurde, sind Typ II- oder Typ III-Gläser.

Das Volumen der bei der Titration benötigten Salzsäure ist ein Maß für die (Alkali-)Auslaugung aus dem erfindungsgemäßen Glas. Aus den nachfolgenden Tabellen sind unter USP die hydrolytischen Beständigkeit der Ausführungsbeispiele (Tabelle 1) und der Vergleichsbeispiele (Tabelle 2) zu entnehmen. Alle genannten Gläser gehören der hydrolytischen Klasse 1 an, die überwiegend nach der USP-Methode bestimmt wurde. Die Vergleichsbeispiele V7 bis V10 entsprechend Bsp. 1 bis Bsp. 4 der DE102010029975A1 enthalten Angaben, die nach der bekannten ISO 719 in µg/g ermittelt wurden. Um die nach unterschiedlichen Methoden bestimmten hydrolytischen Angaben vergleichbar zu machen, ist aus der Angabe "% des Grenzwertes" zu entnehmen, in welchem Bereich innerhalb der hydrolytischen Klasse 1 sich ein Glas befindet. Dieser Wert ist somit ein Qualitätsmerkmal des Glases. Je niedriger er ist, umso besser ist die hydrolytische Beständigkeit des jeweiligen Glases. Somit ist aus den Tabellen klar zu entnehmen, dass die gemäß der Erfindung gefertigten Ausführungsbeispiele eine bessere hydrolytische Beständigkeit aufweisen, als bekannte und teilweise im pharmazeutischen Bereich bereits eingesetzte Neutralgläser. Das bedeutet, dass aus dem erfindungsgemäßen Zirkoniumfreien, Bor-armen Neutralglas weniger Ionen freigesetzt werden, als aus anderen pharmazeutischen Gläsern.

Von den hydrolytischen Klassen sind die Säureklassen nach DIN 12116 und die Laugenklassen nach ISO 695 zu unterscheiden. Insgesamt zeigen die Messwerte der Ausführungsbeispiele, dass das erfindungsgemäße Neutralglas auch bezüglich der Laugen- und Säurebeständigkeit besser ist als aktuelle Pharmagläser.

Bei den Ausführungsbeispielen tritt bis auf ein Beispiel keine Entglasung auf. Die Entglasung, d.h. das Kristallwachstum im Glas (Kristallisationsgeschwindigkeit KG (in µm/min)) wurde nach der bekannten Norm ASTM C829-81 bestimmt. Aufgrund der hohen Kristallisationsbeständigkeit eignen sich die erfindungsgemäßen Gläser für das Ziehen nach dem Danner-Verfahren. Auch der Wärmeausdehnungskoeffizient (CTE, in 10⁻⁶K⁻¹) entspricht den Anforderungen an ein Neutralglas, insbesondere für den Pharmabereich. Ebenso die Transformationstemperatur Tg (°C).

Die Ergebnisse zeigen somit, dass die erfindungsgemäßen Neutralgläser zur bevorzugten Verwendung als Primärpackmittel im Pharmabereich geeignet sind und insbesondere für wässrige oder wasserhaltige Arzneimittel zum Einsatz kommen können. Die in den erfindungsgemäßen Behältnissen aufbewahrten Arzneimittel greifen das Glas nicht in nennenswerter Weise an.

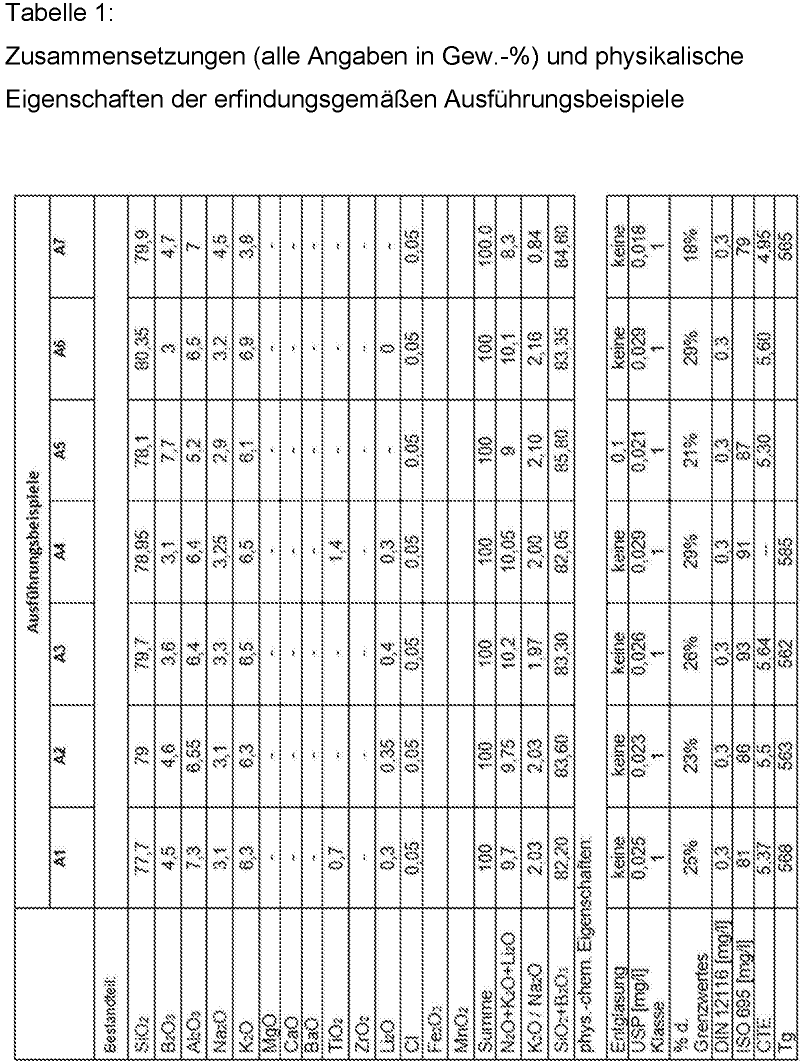

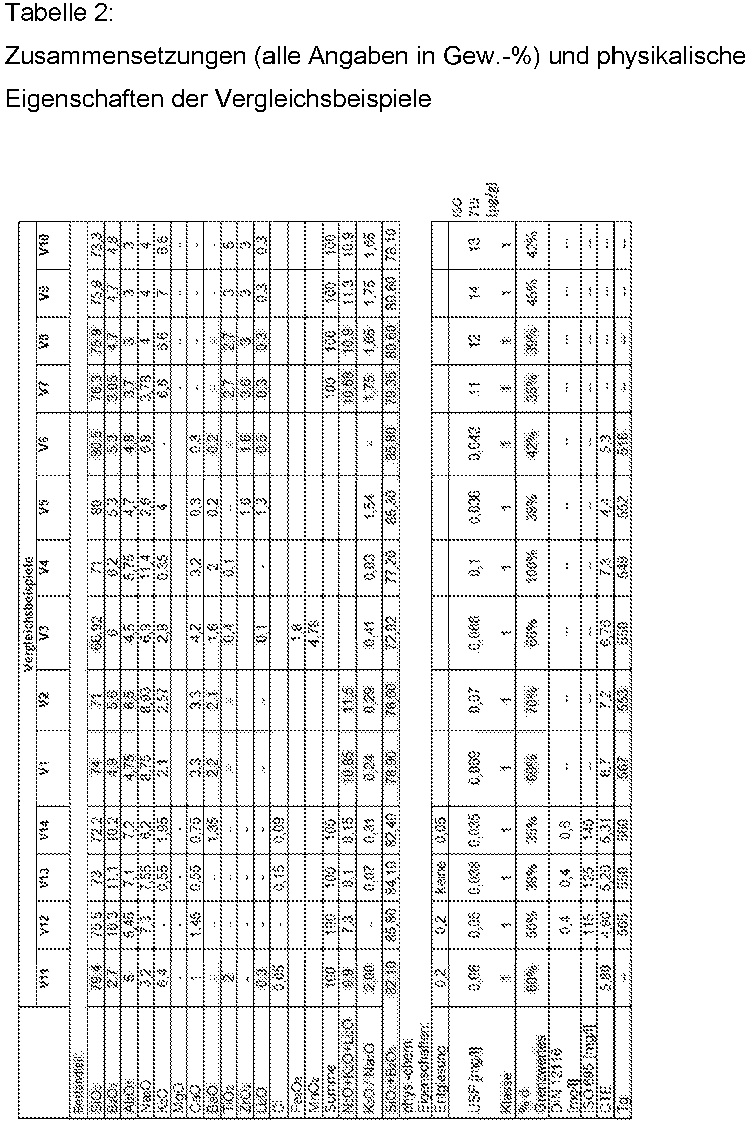

## Patentansprüche

1. Neutralglas, bevorzugt zur Verwendung im Pharmabereich, das frei von Zirkonium ist und die nachfolgenden Komponenten in Gew.-% aufweist:
| | |
|---|---|
| SiO₂ | 72 - 82 |
| B₂O₃ | 3 - 8 |
| Al₂O₃ | 5 - 8 |
| Na₂O | 2,5 - 5,5 |
| K₂O | 3,6 - 8,4 |
| Li₂O | 0 - 0,7 |
| MgO | 0 - 0,7 |
| CaO | 0 - 0,4 |
| TiO₂ | 0 - 5 |
| Li₂O+ Na₂O+ K₂O | 6,8 - 14,6 |
| Verhältnis K₂O/Na₂O | 0,7 - 3,4 |

2. Neutralglas nach Anspruch 1, **dadurch gekennzeichnet, dass** der SiO₂-Gehalt im Bereich von 75 bis 81 Gew.-%, bevorzugt im Bereich von 77 bis 80 Gew.-% liegt.

3. Neutralglas nach Anspruch 1 oder 2, **dadurch gekennzeichnet, dass** der B₂O₃-Gehalt im Bereich von 3 - 6,5 Gew.-%, bevorzugt im Bereich von 3,5 - 5,5 Gew.-% liegt.

4. Neutralglas nach wenigstens einem der Ansprüche 1 bis 3, **dadurch gekennzeichnet, dass** der Al₂O₃-Gehalt 5 bis 7,5 Gew.-% beträgt.

5. Neutralglas nach wenigstens einem der Ansprüche 1 bis 4, **dadurch gekennzeichnet, dass** es kein BaO enthält.

6. Neutralglas nach wenigstens einem der Ansprüche 1 bis 5, **dadurch gekennzeichnet, dass** der Na₂O-Gehalt im Bereich von 2,8 - 4,8 Gew.-% liegt.

7. Neutralglas nach wenigstens einem der Ansprüche 1 bis 6, **dadurch gekennzeichnet, dass** der K₂O-Gehalt im Bereich von 4 - 8 Gew.-%, bevorzugt im Bereich von 4,2 - 7,8 Gew.-% liegt.

8. Neutralglas nach wenigstens einem der Ansprüche 1 bis 7, **dadurch gekennzeichnet, dass** der LiO₂-Gehalt 0 - 0,5 Gew.-% beträgt.

9. Neutralglas nach wenigstens einem der Ansprüche 1 bis 8, **dadurch gekennzeichnet, dass** die Summe der Alkalioxide im Glas zwischen 6,8 und 13,3 Gew.-%, bevorzugt zwischen 7,0 und 12,6 Gew.-% liegt.

10. Neutralglas nach wenigstens einem der Ansprüche 1 bis 9, **dadurch gekennzeichnet, dass** das Verhältnis von K₂O/Na₂O im Bereich von 0,8 bis 2,9, bevorzugt im Bereich von 0,9 bis 2,8 liegt.

11. Neutralglas nach wenigstens einem der Ansprüche 1 bis 10, **dadurch gekennzeichnet, dass** es kein Erdalkalioxid enthält.

12. Verwendung eines Neutralglas nach wenigstens einem der Ansprüche 1 bis 11 zum Herstellen eines gezogenen Rohres.

13. Verwendung eines Neutralglases nach einem der vorangehenden Ansprüche 1 bis 11 im Pharmabereich, insbesondere für Fläschchen, Spritzen, Ampullen und Karpulen.

14. Pharmaprimärpackmittel, **dadurch gekennzeichnet, dass** es aus einem Neutralglas nach mindestens einem der vorangehenden Ansprüche 1 bis 11 besteht.

15. Primärpackmittel, hergestellt aus dem erfindungsgemäßen Neutralglas nach mindestens einem der Ansprüche 1 bis 11, **dadurch gekennzeichnet, dass** es mindestens eine Komponente enthält, ausgewählt aus der Gruppe von Wasser, Wirkstoff, Pufferlösung im pH-Bereich 1 bis 11.

## Claims

1. Neutral glass, preferably for use in the pharmaceutical sector, which is free of zirconium and has the following components in % by weight:
| | |
|---|---|
| SiO₂ | 72 - 82 |
| B₂O₃ | 3 - 8 |
| Al₂O₃ | 5 - 8 |
| Na₂O | 2.5 - 5.5 |
| K₂O | 3.6 - 8.4 |
| Li₂O | 0 - 0.7 |
| MgO | 0 - 0.7 |
| CaO | 0 - 0.4 |
| TiO₂ | 0 - 5 |
| Li₂O+ Na₂O+ K₂O | 6.8 - 14.6 |
| K₂O/Na₂O ratio | 0.7 - 3.4. |

2. Neutral glass according to Claim 1, **characterized in that** the SiO₂ content is in the range from 75% to 81% by weight, preferably in the range from 77% to 80% by weight.

3. Neutral glass according to Claim 1 or 2, **characterized in that** the B₂O₃ content is in the range of 3%-6.5% by weight, preferably in the range of 3.5%-5.5% by weight.

4. Neutral glass according to at least one of Claims 1 to 3, **characterized in that** the Al₂O₃ content is 5% to 7.5% by weight.

5. Neutral glass according to at least one of Claims 1 to 4, **characterized in that** it does not contain any BaO.

6. Neutral glass according to at least one of Claims 1 to 5, **characterized in that** the Na₂O content is in the range of 2.8%-4.8% by weight.

7. Neutral glass according to at least one of Claims 1 to 6, **characterized in that** the K₂O content is in the range of 4%-8% by weight, preferably in the range of 4.2%-7.8% by weight.

8. Neutral glass according to at least one of Claims 1 to 7, **characterized in that** the LiO₂ content is 0%-0.5% by weight.

9. Neutral glass according to at least one of Claims 1 to 8, **characterized in that** the sum total of the alkali metal oxides in the glass is between 6.8% and 13.3% by weight, preferably between 7.0% and 12.6% by weight.

10. Neutral glass according to at least one of Claims 1 to 9, **characterized in that** the ratio of K₂O/Na₂O is in the range from 0.8 to 2.9, preferably in the range from 0.9 to 2.8.

11. Neutral glass according to at least one of Claims 1 to 10, **characterized in that** it does not contain any alkaline earth metal oxide.

12. Use of a neutral glass according to at least one of Claims 1 to 11 for production of a drawn tube.

13. Use of a neutral glass according to any of the preceding Claims 1 to 11 in the pharmaceutical sector, especially for vials, syringes, ampoules and carpules.

14. Primary pharmaceutical packaging, **characterized in that** it consists of a neutral glass according to at least one of the preceding Claims 1 to 11.

15. Primary packaging produced from the inventive neutral glass according to at least one of Claims 1 to 11, **characterized in that** it comprises at least one component selected from the group of water, active ingredient, buffer solution in the pH range of 1 to 11.

## Revendications

1. Verre neutre, de préférence destiné à une utilisation dans le domaine pharmaceutique, qui est exempt de zirconium et comprend les composants suivants en % en poids :
| | |
|---|---|
| SiO₂ | 72 à 82 |
| B₂O₃ | 3 à 8 |
| Al₂O₃ | 5 à 8 |
| Na₂O | 2,5 à 5,5 |
| K₂O | 3, 6 à 8,4 |
| Li₂O | 0 à 0,07 |
| MgO | 0 à 0,7 |
| CaO | 0 à 0,4 |
| TiO₂ | 0 à 5 |
| Li₂O + Na₂O + K₂O | 6,8 à 14,6 |
| rapport K₂O/Na₂O | 0,7 à 3,4. |

2. Verre neutre selon la revendication 1, **caractérisé en ce que** la teneur en SiO₂ se situe dans la plage allant de 75 à 81 % en poids, de préférence dans la plage allant de 77 à 80 % en poids.

3. Verre neutre selon la revendication 1 ou 2, **caractérisé en ce que** la teneur en B₂O₃ se situe dans la plage allant de 3 à 6,5 % en poids, de préférence dans la plage allant de 3,5 à 5,5 % en poids.

4. Verre neutre selon au moins l'une quelconque des revendications 1 à 3, **caractérisé en ce que** la teneur en Al₂O₃ est de 5 à 7,5 % en poids.

5. Verre neutre selon au moins l'une quelconque des revendications 1 à 4, **caractérisé en ce qu'**il ne contient pas de BaO.

6. Verre neutre selon au moins l'une quelconque des revendications 1 à 5, **caractérisé en ce que** la teneur en Na₂O se situe dans la plage allant de 2,8 à 4,8 % en poids.

7. Verre neutre selon au moins l'une quelconque des revendications 1 à 6, **caractérisé en ce que** la teneur en K₂O se situe dans la plage allant de 4 à 8 % en poids, de préférence dans la plage allant de 4,2 à 7,8 % en poids.

8. Verre neutre selon au moins l'une quelconque des revendications 1 à 7, **caractérisé en ce que** la teneur en LiO₂ est de 0 à 0,5 % en poids.

9. Verre neutre selon au moins l'une quelconque des revendications 1 à 8, **caractérisé en ce que** la somme des oxydes alcalins dans le verre est comprise entre 6,8 et 13,3 % en poids, de préférence entre 7,0 et 12,6 % en poids.

10. Verre neutre selon au moins l'une quelconque des revendications 1 à 9, **caractérisé en ce que** le rapport K₂O/Na₂O se situe dans la plage allant de 0,8 à 2,9, de préférence dans la plage allant de 0,9 à 2,8.

11. Verre neutre selon au moins l'une quelconque des revendications 1 à 10, **caractérisé en ce qu'**il ne contient pas d'oxyde alcalino-terreux.

12. Utilisation d'un verre neutre selon au moins l'une quelconque des revendications 1 à 11 pour la fabrication d'un tube étiré.

13. Utilisation d'un verre neutre selon l'une quelconque des revendications 1 à 11 dans le domaine pharmaceutique, notamment pour des bouteilles, des seringues, des ampoules et des cartouches.

14. Emballage primaire pharmaceutique, **caractérisé en ce qu'**il est constitué par un verre neutre selon au moins l'une quelconque des revendications 1 à 11 précédentes.

15. Emballage primaire, fabriqué à partir du verre neutre selon l'invention selon au moins l'une quelconque des revendications 1 à 11, **caractérisé en ce qu'**il contient au moins un composant choisi dans le groupe constitué par l'eau, un agent actif, une solution tampon dans la plage de pH allant de 1 à 11.
